# EUROPEAN PATENT SPECIFICATION

(11) **EP 1 738 789 B1**
(45) Date of publication and mention of the grant of the patent: **20.04.2011**
(21) Application number: 06013710.6
(22) Date of filing: 03.07.2006
(51) Int. Cl.: A61M 16/04, A61B 1/267

(54) **Endotracheal video device**
Endotracheale Videovorrichtung
Dispositif vidéo endotrachéal

(30) Priority: 01.07.2005 US 174052
(43) Date of publication of application: 03.01.2007
(73) Proprietor: Karl Storz GmbH & Co. KG, 78532 Tuttlingen (DE)
(72) Inventor: Boedeker, Ben, 68135 Omaha (US); Berci, George, Los Angeles, CA 90025 (US); Kaplan, Marshal B., CA 90210 Beverly Hills (US); Barry, James P., 01507 Charlton (US); Chatenever, David, Santa Barbara, CA 93105 (US); Irion, Klaus M., Dr.-Ing., 78576 Emmingen-Liptingen (DE); Ehrhardt, André, 78573 Wurmlingen (DE); Rudischhauser, Jürgen, 78532 Tuttlingen (DE); Mattsson-Boze, Daniel, Sacramento, CA 95835 (US)
(74) Representative: Weller, Wolfgang

(56) References cited:
- WO-A-02/11608
- WO-A-98/19589
- WO-A-03/079889
- US-A- 5 183 031
- US-A- 5 425 356
- US-A- 5 840 013
- US-A- 5 879 289
- US-A1- 2002 022 769
- US-A1- 2003 088 156

## Description

### Field of the Invention

The invention relates to a medical device having an endotracheal device and a supra-laryngeal mask, wherein said endotracheal device is attachable to a camera.

### Background of the Invention

In the United States, approximately 20 million patients are operated on and anesthetized each year. Approximately 50 % of surgeries are performed using general anesthesia, which means the patient is put to sleep and the ventilation and other physiological function are monitored. While anesthetized, the patient's breathing functions are temporarily disabled.
Ventilation is therefore supplied to the patient by the anesthesiologist during the procedure.

Ventilation should be typically controlled mainly in the elderly, difficult cases with severe underlying disease, or those with severe trauma by introducing a tube into the windpipe (trachea), and the respiratory function are controlled by an automatic ventilator. In a small percentage, (5 - 10%) of patients who have to undergo general anesthesia, a Laryngeal Mask (LMA) intubation is employed. A special group of patients are those with emergency situations. A LMA may include a Supra Laryngeal Device (SLD), which typically includes a flexible tube (18 - 20 mm OD), which has an attached small inflatable mask at the distal end. This device is introduced through the mouth into the hypo-pharynx. The inflatable mask is inflated and the esophagus is blocked to avoid air-oxygen inflation in the gastro-intestinal organs. The open part of the mask is positioned opposite the vocal cords. The inflated mask is blocking the air insufflation into the esophagus and stomach. The ventilation is provided through a tube connecting the opening of the mask into the trachea. The proximal end of the tube is connected to a ventilator, which is adjusted according to the physiological parameters of the patient's needs.

Ventilation is typically provided through an endotracheal tube. This tube is inserted into the trachea, and it is closed against the wall of the trachea by an inflatable cuff. The insertion of this tube involves risks that the anesthesiologist seeks to avoid or at least minimize. It is estimated that between one in 6,000 to one in 8,000 general anesthesia procedures result in death. There are of course many causes but of these it is estimated that about one third of them are caused by the intubation procedure.

The foremost obstacles encountered by the anesthesiologist include; the remoteness of the location where the tube is to be positioned, the consequent restriction of view as the tube is inserted, variations and anomalies in the anatomy of the patients, an uncomfortable and unnatural position for the anesthesiologist while holding the instrument, the potential need to change blades during the procedure, and the necessity for rapid intubation.

The positioning of the LMA, of which there are numerous types, is typically performed blindly. The proper position can be assessed by the exhaled CO₂ content (capnometer), but this is typically only available in operating rooms.

It should be noted that when the tube is inserted, the patient is asleep hyperoxygenated and then paralyzed for the procedure, and therefore not breathing. In addition, the ventilator is not yet in operation. This gives the anesthesiologist only about two minutes in which to intubate the patient, inflate the cuff, and start ventilation. If he is delayed because of unsuccessful attempts, he must stop, apply a ventilation mask to the patient, supply oxygen for a time through the mask, remove the mask, adjust medication if necessary, and then start over again. This delays the operation and extends the patient's time under anesthesia. This extension of time while under anesthesia may have very serious consequences, especially for elderly patients.

With the advent of endoscopic equipment and small cameras, instrumentation has been improved to the extent that it can enable viewing of the cords and larynx on a video screen thereby facilitating the intubation of the patient in a relatively quick and safe manner. However, conventional instrumentation may be further improved such that the laryngoscope is easier to use, thereby reducing the time involved for instance, to change blades or attach and detach peripheral components.

Endoscopes are now widely used in minimally invasive surgery. Endoscopes typically contain a light guiding system, usually in the form of fiber optic cables, in order to bring light to the surgical area. The light guiding system typically extends through the handle of the laryngoscope and through a guide tube located in the blade so as to position the light guiding system to illuminate the area ahead of the blade. Endoscopes also typically contain an image guiding system, for example in the form of a rigid rod lens system, arranged in the shaft of the endoscope. The image guiding system can also be configured as an ordered, flexible fiber optic bundle. The image guiding system is utilized to transmit reflected light from the area ahead of the blade to a camera. The camera, attached at the proximal end of the endoscope, usually contains a CCD (charge coupled device) sensor, in the form of a light-sensitive chip that converts the optical signals into electrical signals that are conveyed from the image-sensing camera module to a remotely located image processing system. The image guide typically extends from the distal end of the blade through the guide tube and then through the handle of the laryngoscope.

Typically, the combination light guiding system and image guiding system are permanently attached to the handle and are continuous, extending from the distal end of the blade, through the handle of the laryngoscope and to the camera for the image guiding system, and to the light source for the light guiding system. Therefore, the light guiding system and image guiding system extending from the handle of the laryngoscope for insertion into the guide tube of the blade typically comprise flexible coherent fiber optic bundles. However, when changing blades, the bundle must be carefully inserted or withdrawn from the opening of the guide tube at the proximal end of the blade. This may take an unacceptable amount time for the physician to thread the bundle into the tube if the blade must be changed in the middle of the intubation process.

The light and image guiding systems have typically been permanently attached to the handle to ensure the system will reliably transmit the illuminating light and reflected images. To utilize a detachably connectable light and image guiding system, the attachment means would have to rigidly hold the member in place such that the light and image guiding systems did not become misaligned. In addition, the attachment means must be easy and quick to operate, making it possible to perform the coupling procedure with as little close attention as possible, but nevertheless reliably. Provision must therefore be made for the coupling elements to be keyed to each other so that the coupling cannot be incorrectly joined and so that close attention by the operation is not required.

In addition, the flexible bundles may easily be damaged and will wear over time, degrading or rendering the system inoperable. As a visual inspection of the device often will not indicate whether the bundles are damaged, it is conceivable that a physician may obtain a damaged or malfunctioning laryngoscope not realizing that it is damaged. The time involved with determining that the instrument is malfunctioning, withdrawing it, finding another laryngoscope, and then intubating the patient may have severe adverse effects upon the patient under anesthesia.

Further, laryngoscopes, as with most medical equipment, must be sterilized after use. Because the light and image guiding systems are permanently attached to the handle, they are exposed to extremely high temperatures, which also cause wear and/or failure of the flexible bundles. Also, because the light and image guiding systems are subjected to the sterilization process with the handle and blades, the handle must be hermetically sealed which may greatly add to the cost in manufacturing such a device.

WO 03/079889 A1 discloses a laryngoscope with an image sensor, wherein an imaging device comprising the image sensor being attached to the blade of a laryngoscope which is flexible in one embodiment of the laryngoscope.

US 2002/0022769 E1 discloses a portable video laryngoscope that comprises a probe having a controllable pivot point.

US 5,872,298 A discloses a portable, handheld endoscopic camera to which distal end different interchangeable probes can be attached.

One of these interchangeable probes comprises a fiber optic cable through which the light and image information are sent, a neck for the attachment to the distal end of the handheld endoscopic camera, and a part along its length having accordion-shaped ribs which allow an individual bending of this portion by an operator into a desired shape.

US 5,840,018 A describes an endoscope that can be rigid, flexible, partially rigid or partially flexible or adjustable and can include a CCD sensor.

WO 02/11608 A2 discloses an intubation instrument which works similar to a laryngoscope but is constructed differently, and comprises digital imaging capabilities.

It is therefore desired to provide an improved endotracheal device, specifically for use with a LMA that allows for visual feedback to a user during insertion.

It is further desired to provide an improved portable endotracheal device for field use that allows a medical worker located remotely from the patient to view images of the insertion by a user.

It is still further desired to provide an improved portable endotracheal video device that may be detached from for example, a disposable LMA and an image light guide system to allow for sterilization and the reuse with the supra laryngeal device (SLD).

### Summary of the Invention

These and other objects are achieved by providing a medical device having an endotracheal device and a supra-laryngeal mask, wherein said endotracheal device is attachable to a camera and comprises a light guide, an image guide, a first connector, said first connector terminating the light guide and the image guide and being engageable with a second connector on the camera, and an outer housing enclosing said light and said image guides, said outer housing having a flexible portion that may be bent by a user, and a rigid portion that substantially maintains a rigid preformed shape, and wherein said supra-laryngeal mask comprises a sheath attached to and substantially following a curvature of said supra-laryngeal mask, said sheath having the endotracheal device inserted therein, and wherein a securing clip for affixing the endotracheal device to the supra-laryngeal mask, wherein said securing clip fastens between the supra-laryngeal mask and the rigid portion of the outer housing of the endotracheal device.

The endotracheal device may in one embodiment be detachably connectable to or insertable in the LMA or alternatively, may be integrally formed in or with the LMA.

The endotracheal device may comprise flexible image and light guides, which may advantageously continue into a rigid performed tube with an outside diameter of for example, approximately 3mm. The endotracheal device may be attached to the LMA via an additional tube attached to the LMA or may be inserted into a cavity formed in the LMA for receiving the endotracheal device. The tip of the endotracheal device may be positioned in front of the mask opening toward the vocal cord for displaying the positioning of the mask on a video screen. Near the top of the tube of the LMA, the light and image guides may continue in a rigid tube and connected to a coupling mechanism. The coupling mechanism that connects the endotracheal device, for example a camera.

Accordingly, the coupling mechanism is provided with a first cylindrical stem of specific diameter and specific length, in whose interior is received a proximal end segment of the light guiding system, and which projects from one coupling end of the endotracheal device in the coupling direction. Further, a second cylindrical stem is provided whose length and diameter are greater than the length and diameter of the first stem, having a proximal end segment of the image guiding system being received in the interior of the second stem, and which projects from one coupling end of the endotracheal device in the coupling direction. The second stem coacts with an interlock system arranged in the laryngoscope handle forming a rigid mechanical coupling, the first and second stems extending at a distance next to one another. Complementary receptacles corresponding to the two stems, into which the stems penetrate, are provided in the laryngoscope handle. The base of the receptacle into which the second stem penetrates is optically connected to the camera, and the receptacle in which the shorter first stem is receivable is connected to the light source.

The mechanical, light-guiding, and image-guiding coupling is accomplished by way of a single simple linear displacement operation, in which specifically the two stems are pushed into the corresponding receptacles of, for example, a camera or a laryngoscope handle. Because one of the two stems is thicker and longer than the other, incorrect (i.e. reversed) insertion is not possible. Because the thicker stem is also simultaneously the longer one, it is possible, without undue attention, to feel for the correspondingly larger receptacle on the camera module with this thicker and longer stem, and then to close the coupling with an insertion movement. Incorrect attachment is thus no longer possible, since the thicker and longer stem cannot be attached to the smaller-diameter receptacle for the smaller and shorter stem.

The mechanical interlock or coupling is affected simultaneously with this insertion. Because the larger stem is also the longer stem, and it carries the image guiding system, the image guiding connection occurs at an axial spacing from the light-guiding connection. This feature has the advantage that any stray light that might emerge from the light connection cannot directly come into contact with the image-guiding connecting point located at an axial distance therefrom. The disadvantages of connecting image and light at the same level, or those, for example, of a coaxial arrangement, are thus eliminated.

Because the coupling mechanism is keyed, the user can therefore, for example, sense the camera or laryngoscope handle and its precise grasped position in the coupling region with one hand, and with the other hand can easily sense the light and image guiding attachment and its grasped position as well, so that the two elements to be coupled can then be inserted into one another without visual contact.

An interlock system is displaceable transversely to the coupling direction that can be engaged into a recess on the second stem. This feature has the advantage that in order to close and/or release the coupling, the locking element is displaced transversely to the coupling direction and is engaged into or disengaged from the recess on the second stem. These are all procedures that can be controlled, without visual contact, with the fingers of one hand; the snapping of the locking element into and out of the recess on the stem indicates to the operator whether the coupling is closed or open. If the locking element needs to be pushed into the recess, for example to close the coupling, this can be done by simply inserting the stems into their corresponding receptacles; precise locking can be ascertained by an audible sound that the locking element has been engaged. The locking element may comprise for instance, ball catches, hooks, snap lugs, or the like.

The locking element is acted upon by the force of a spring, and radially projects into the receptacle for the second stem. This is advantageous because, the force of the spring presses the locking element into a defined position, and the coupling may be disengaged by the application of a force opposite the coupling direction, namely withdrawing the stems from their respective receptacles. These are all procedures that can be sensed and controlled with the hand's sense of touch, so that no visual attention or observation is necessary when closing and opening the coupling.

The second stem may have a conical segment at the end that is followed by an undercut. The conical segment constitutes an insertion aid upon insertion of the stem into the receptacle, so that exact insertion is guaranteed with even approximate placement. At the same time, the conical surface can be utilized to displace the locking element radially upon insertion.

In addition, the undercut in the second stem may be configured as an annular groove. This forms a relatively large engagement surface with the locking element, so that the mechanical forces acting on the coupling will be dispersed over the entire area, which contributes to mechanical stability and less wear through use.

In addition, the first and second stems along with the receptacles receiving them each have a window. The windows thereby provide a sealed closure for the light and image guiding systems.

The endotracheal device may in one advantageous embodiment, be provided entirely or in part with a stainless steel outer casing, or some other suitable rigid enclosure, for protecting the endotracheal device. As the endotracheal device is detachable from the camera or handle, only the endotracheal device need be subjected to sterilization or can be disposable.

The camera may be provided with a built in light transmitter and optical lens system that is coupled to the endotracheal device by means of the coupling mechanism previously described. The camera may be advantageously used as a handle to position or reposition the SLD to an optimal position during insertion and use.

In the case where a patient has suffered a severe trauma, the SLD may be utilized by semi-trained Medic Corpsman or first-aid personnel in a field setting. The LMA may be provided as a disposable device, whereas the video-image light guide may be re-sterilized for future use.

As the Medi-Corp or first-aid personnel have limited training, it is contemplated the video image may be transmitted via for example, a telemetering device to a remote station and the procedure may be directed by a well-trained doctor in the remote field hospital or an emergency medical physician. In this manner, a relatively large number of individuals with limited training may be able to perform medical procedures with a relatively high degree of accuracy, even a relatively difficult procedure, under the direct supervision of highly trained medical personnel. This is highly desirable on for example, a battle field or in an area where a catastrophic event has occurred.

The endotracheal device with a SLD is attachable to a camera and comprises a light guide, an image guide, and a first connector associated with the endotracheal device, the first connector terminating the light guide and the image guide and engagable with a second connector on the camera. The device further comprises, an outer housing enclosing the light and image guides. The device is provided such that the outer housing has a flexible portion that may be bent by a user, and a rigid portion that substantially maintains a rigid preformed shape.

In another advantageous embodiment the device further comprises a camera for generating image data, the camera having a second connector that is engagable with the first connector along a path of movement.

outer housing has a flexible portion that may be bent by a user, and a rigid portion that substantially maintains a rigid preformed shape, and the endotracheal device connector is engagable with the camera connector along a path of movement.

It is understood that the features mentioned above and those yet to be explained below can be used not only in the respective combinations indicted, but also in other combinations or by themselves, without leaving the context of the present invention.

### Brief Description Of The Drawings

Figure 1 is an illustration of the video laryngoscope with a curved blade and the endotracheal device engaged with the handle;

Figure 2 is an illustration of a curved blade detached from the handle;

Figure 3 is a perspective view of the handle depicting the housing containing the receptacles for the endotracheal device;

Figure 4 is an illustration of the curved endotracheal device as detached from the handle;

Figure 5 is a cross-sectional view of the housing containing the receptacles for the endotracheal device;

Figure 6 is a cross-sectional view of the coupling element utilized in conjunction with the endotracheal device;

Figure 7 is an illustration of the video laryngoscope with a straight blade and the endotracheal device engaged with the handle;

Figure 8 is an illustration of a camera having light and image guiding receptacles at one end and light and image cables connected at the other end;

Figure 9 is an illustration of a Laryngeal Mask (LMA);

Figure 10 is an illustration of the LMA according to Figure 9 including an exterior attached guide tube;

Figure 11 is an illustration of the image light guide with the optical coupler device according to one advantageous embodiment of the present invention; and

Figure 12 is an illustration according to Figure 11 illustrating one advantageous embodiment of the connection of the endotracheal device with the LMA.

### Detailed Description Of The Drawings

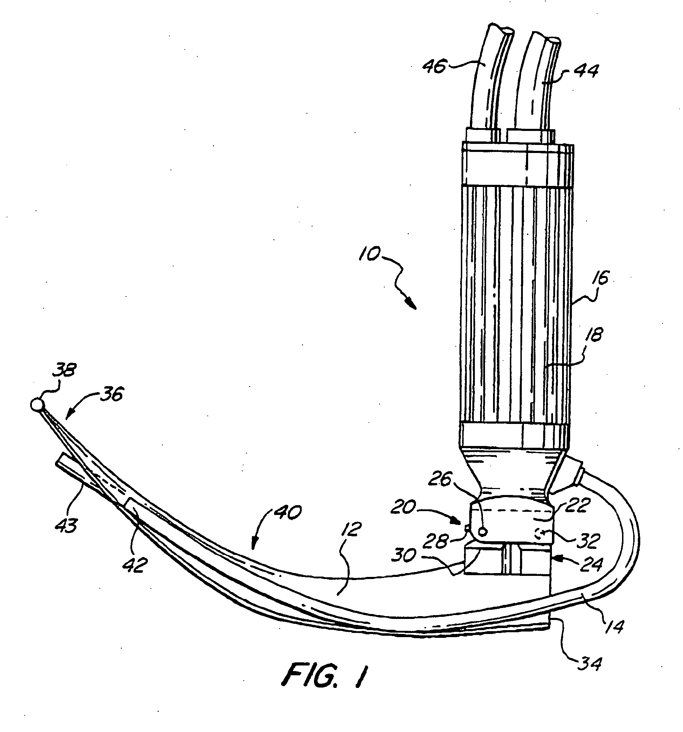

The video laryngoscope 10, along with the attached blade 12 and endotracheal device 14 is illustrated in Figure 1.

The handle 16 is typically cylindrical with a knurled outer surface 18 thereby facilitating a secure gripping surface. As is shown in Figure 1, the handle 16 is detachably joined to a blade 12, which in this instance is curved, by a joinder 20.

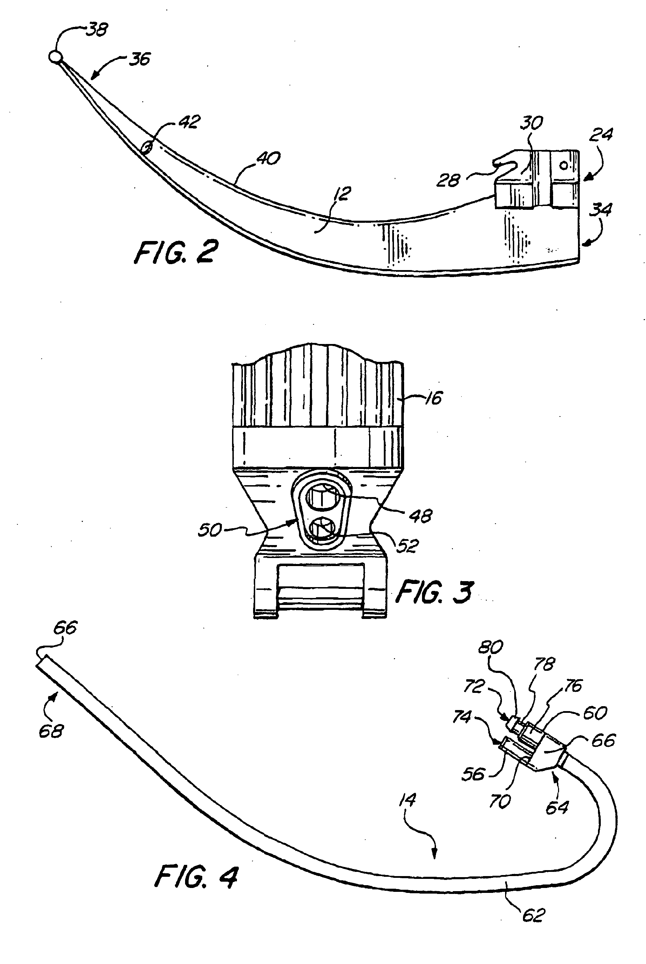

The joinder 20 includes a pair of conventional hinge socket 22 and connector 24 respectively mounted to the lower end of the handle 16 and to a proximal end 34 of the blade 12. Socket 22 further includes a crossbar 26. Connector 24 includes a hook 28 in a block 30 that fits into socket 22 and is more clearly seen in Figure 2. The hook 28 engages the crossbar 26, and the handle 16 is rotated 90 degrees so that the blade will be rigidly held to the handle 16. This is a common joinder 20 used in this type of instrumentation and is useful for all blade forms, of which the two illustrated forms are merely examples. A ball detent 32 detachably retains the handle 16 and blade 12 together and erect in the assembled configuration, The assembled instrument is rigid during the procedure.

Blade 12 has a distal end 36 which may be smoothed by a bulb-like edge 38. It has a curved top surface 40 extending from the distal end 38 toward the proximal end 34. This top surface 40 is used to elevate the tongue and permit the visualization of the vocal cords beneath it.

Referring back to Figures 1 and 2, blade 12 additionally includes an hole 42 at the distal end 36 of the blade 12. The hole 42 is designed to allow passage of a distal end 43 of endotracheal device 14.

The handle is provided with means for obtaining an image of the field located beyond the tip of the blade 12, and for providing illuminating light to that field. In one embodiment, a camera (not shown) is mounted in a chamber inside the handle 16.

An image cable 44 to conduct image data from the camera exits from the top of the handle 16. It is connected to a video set (not shown), which provides data for an image on a video screen (not shown), for observation by the anesthesiologist. In addition, in some embodiments an illumination cable 46 conducts illuminating light to the handle 16.

In one embodiment, light for illumination of the field ahead of the distal end 36 of blade 12 is obtained from a separate light source (not shown) that can be placed in any convenient nearby location. An illumination cable 46, which may comprise a fiber optic bundle extends from the light source (not shown) to the handle 16. The illumination cable 46 need not be coherent, because it does not transmit an image - it transmits only illuminating light. Both the image cable 44 and the illumination cable 46 may enter the top of handle 16.

The actual construction of image cable 44 depends on the arrangement of the camera. In a preferred embodiment, the camera (not shown), customarily a CCD chip, is mounted in the handle 16. In this case, the image cable 44 comprises a electrical cable, which extends from the camera output (not shown), out the top of the handle and to a video display (not shown). The image guide extending through the endotracheal device 14, through the handle 16 and terminating at the camera input (not shown) comprises a bundle of coherent fiber optic cables to transmit the reflected light from the area ahead of the blade 12 to the camera (not shown).

In an alternative embodiment, the camera (not shown) may be located remotely from the video laryngoscope 10. In this case, the image guiding cable 44 would comprise a bundle of coherent fiber optic cables extending through the endotracheal device 14, through the handle 16 and terminating at the camera, which is located remotely from the video laryngoscope 10.

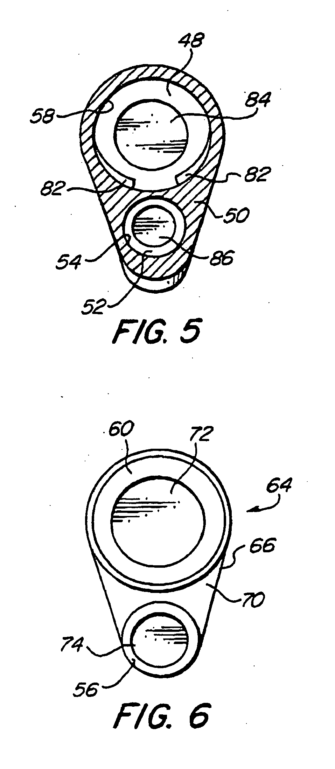

The light guiding system receptacle 52 and the image guiding system receptacle 48 are both contained in housing 50 as illustrated in Figures 3 and 5. The light guiding system receptacle 52 has an inner surface 54 defining a cross-sectional diameter of the receptacle opening corresponding to a diameter of a first stem 56 shown in Figures 4, 5 and 6. Further, the image guiding system receptacle 48 has an inner surface 58 defining a cross-sectional diameter of the receptacle opening corresponding to a diameter of a second stem 60 also shown in Figures 4, 5 and 6.

Figure 4 shows the endotracheal device 14 detached from the handle 16. The endotracheal device 14 may in one advantageous embodiment comprises a rigid curved shaft 62 and a coupling element 64. The coupling element 64 further comprises a first stem 56 and a second stem 60 along with a housing 66. The rigid curved shaft 62 is preferably made of stainless steel but may be manufactured on any rigid non-corroding material. The rigid curved shaft contains both, the light guiding cable 46 for transmitting illuminating light ahead of the distal end 36 of the blade 12, and the image guiding cable 44 for receiving the reflected light and transmitting it to the camera (not shown) located in the handle 16. The rigid curved shaft 62 also has a window 66, located at the distal end 68, which acts to seal the endotracheal device 14. While it is contemplated that the light guiding cable 46 and the image guiding cable 44 are detachably connectable with the handle 16 via the coupling element 64, it is further contemplated that the light guiding cable 46 and the image guiding cable 44 associated with blade 40 may or may not be detachable from blade 40.

Referring back to the coupling element 64, an approximately cylindrical second stem 60, protrudes at one coupling end 70 of housing 66. The length and inside diameter of image guiding system receptacle 48 are selected so that second stem 60 can be received snugly therein. A window 72 is provided at the end of the second stem 60 to provide a seal for the image guiding cable 44. A first approximately cylindrical stem 56 extends from one coupling end 70 of housing 66 parallel to second stem 60. The first stem 56 is smaller in diameter and length than the second stem 60. Also window 74 is provided at the end of the first stem 56 to provide a seal for the light guiding cable 46.

Second stem 60, comprises a cylindrical segment 76, and annular groove 78, and a terminal conical segment 80. Both stems 56 and 60 extend in a coupling direction to mate with receptacles 52 and 48 respectively.

A locking element 82, displaceable radially with respect to the coupling direction, is located in housing 50. Locking element 82 may be approximately the shape of a two-tined fork that is bent inward in a circular shape at the outer end, the radius of curvature corresponding approximately to the radius of curvature of image guiding system receptacle 48. The outer ends of locking element 82 project slightly into image guiding system receptacle 48 as shown in Figure 5.

Conical segment 80 of second stem 60 thereby encounters the ends of locking element 82 projecting into image guiding system receptacle 48 and displaces them radially outward.

When second stem 60 has been pushed into image guiding system receptacle 48 to the point that the ends of locking element 82 come to rest at the level of annular groove 78, they snap into annular groove 78.

In this position the coupling is now closed, i.e. coupling system 10 is coupled and mechanically interlocked. In this state, window 72 of second stem 60 and window 84 in the base of image guiding system receptacle 48 lie congruently with one another, thus creating an image-guiding coupling. Window 74 of first stem 56 comes to rest in front of window 86 of light guiding system receptacle 52, so that a light-guiding coupling is also created.

All that is necessary to release the coupling is withdraw the endotracheal device 14 outward with enough force to overcome the locking element 82 as engaged in annular groove 78.

It is now seen that joining the blade 12, which may integrally include the endotracheal device 14, to the handle 16 is a swift process. The portions of the joinder 20 are engaged and the handle 16 is then rotated, locking the blade 12 into place. The endotracheal device 14 may further engage with coupling element 64 in a coupling direction.

Having a detachably connectable endotracheal device 14 means that the endotracheal device 14 may be detached from the handle 16 for sterilization. This further means that the handle 16 must no longer be hermetically sealed for sterilization. This provides the distinct advantage of lowering the cost involved with manufacturing the handle 16 as now they do not have to be subjected to the extremely high temperatures associated with the sterilization process. Further, any electronic components located in the handle 16 will no longer be subjected to the high temperatures of sterilization, which could prematurely age or damage them. While it is contemplated that endotracheal device 14 is detachably connectable with the handle 16, it is further contemplated that endotracheal device 14 associated with blade 40 may or may not be detachable from blade 40. It is further contemplated that while both light guiding system receptacle 52 and the image guiding system receptacle 48 are illustrated in the Figures as located on the side housing 50, it understood that the receptacles may be located at any convenient on housing 50 to match up with blade 40, such as for instance, when endotracheal device 14 is provided integral with blade 40.

Instead of the camera and illumination arrangements already described, there are other alternatives, which can be used in any combination.

For example, instead of employing a separate light source (not shown), a battery and light bulb may be contained in the handle 16, and the light from this bulb focused onto light guiding cable 46. This eliminates the need for a fiber optic bundle from a light source, and also eliminates the separate light source itself.

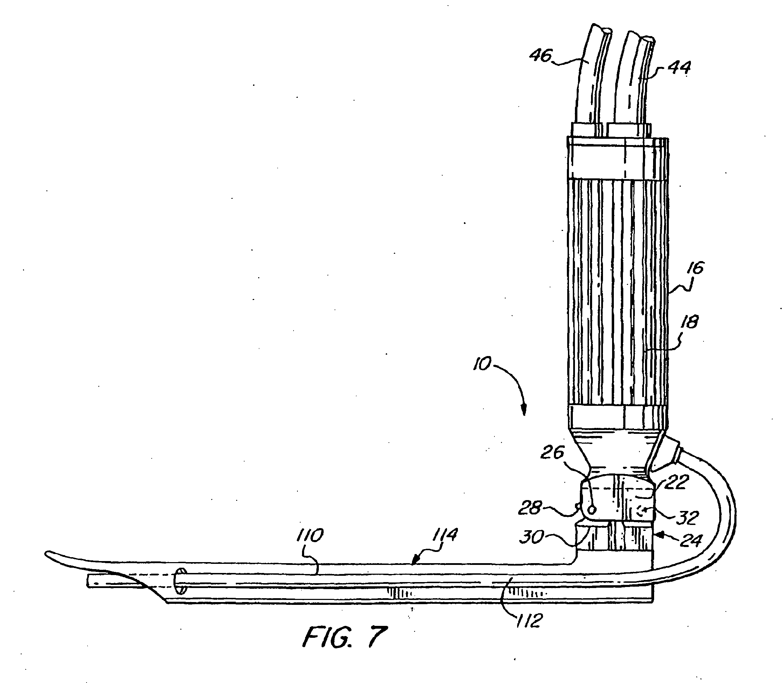

The video laryngoscope 10, along with the attached blade 110 and endotracheal device 112 is illustrated in Figure 7.

The blade 12 illustrated in Figures 1 and 2 is a curved blade 12, which is used to elevate the patients tongue in some circumstances. It is the well-known Mcintosh blade 12. However, a different blade 110 for a different anatomical configuration is a straight blade 110 adapted for use in other circumstances. It is illustrated in Figure 7. This is the well-known Foregger-Magill blade 110. These are the two most common blade shapes. Their configuration is not a limitation on the invention. The configurations of this handle 16 and these blades are completely conventional. They are standard equipment utilized by anesthesiologists trained to intubate the trachea. An advantage of this invention is that it does not require any additional training or re-training of anesthesiologists who have used these well known blades and will utilized them in the future.

The curved blade 12 illustrated in Figures 1 and 2 differs from the straight blade 110 illustrated in Figure 7 only by its shape. The straight blade 110 in Figure 7 has a straight upper surface 114 instead of a curved surface 40 for use when such a surface is preferred for lifting the tongue of the individual patient. In all cases the objective is to lift the tongue to permit visualization of the vocal cords and to enable the endotracheal tube to be accurately placed without harming surrounding tissue in the process.

In addition, the endotracheal device 112 as shown in Figure 7, differs from the endotracheal device 14 as depicted in Figures 1 and 4 only by its shape. The endotracheal device 14 is curved to match the curved blade 12 as depicted in Figures 1 and 4, whereas the endotracheal device 112 is a straighter configuration corresponding to the straight blade 110 as shown in Figure 7.

As the handle 16, the blade 110 and the endotracheal device 112 operate in the same manner as the aforedescribed curved blade 12 and endotracheal device 14, they will not be re-described.

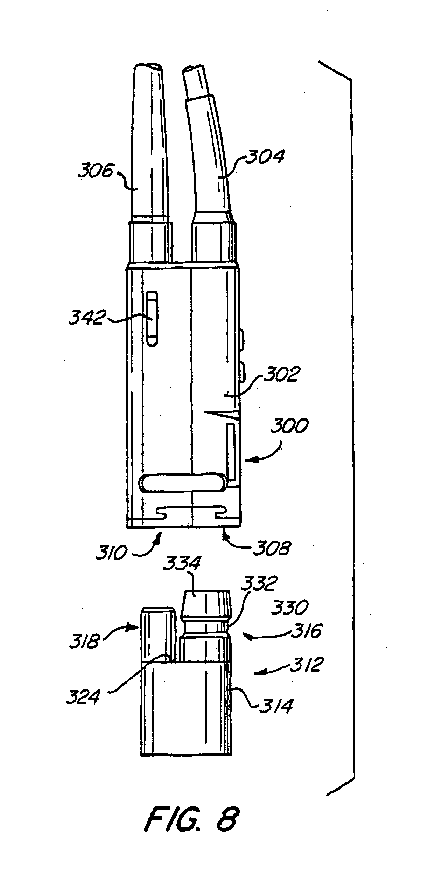

A camera 300 according to one advantageous embodiment of the present invention is illustrated in Figure 8. The camera 300, customarily comprising a CCD chip, has a housing 302 that is generally rectangular but may be any shape. At one end, an image guiding cable 304 and light guiding cable 306 are connected to the camera 300.

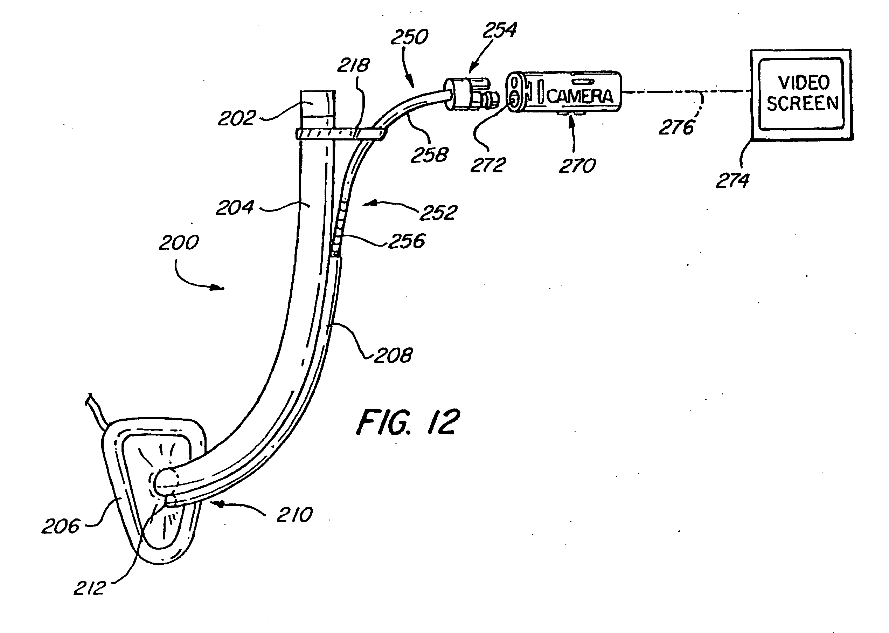

Image guiding cable 304 may comprise an electrical cable, which extends from the camera output to a video screen 274 (FIG. 12). Image guiding cable 304, may utilize any suitable format and protocol for the transmission of video images. Image guiding cable 304 may be permanently attached to camera 300 as illustrated in Figure 8, or may be detachably connectable to camera 300.

Also shown attached to camera 300 is light guiding cable 306. Light guiding cable 306 may comprise any suitable cable (typically fiber optic) for the transmission of illuminating light from an illumination source to a location to be illuminated. Light guiding cable 306 may comprise coherent or non-coherent fiber optic cables and be permanently attached to camera 300 as illustrated in Figure 8, or may be detachably connectable to camera 300. While image guiding cable 304 and light guiding cable 306 are each shown separate from each other, it is contemplated that both may be enclosed in a protective jacket as a single cable.

At the end opposite to where image guiding cable 304 and light guiding cable 306 attach to camera 300, image guiding receptacle 308 and light guiding receptacle 310 are provided. These receptacles are more readily seen as previously illustrated in Figure 5.

It can be seen from Figure 8 that housing 302 of camera 300 is contoured, which allows easy gripping and manipulation of camera 300 by a user even without having to look at the device. Rather, based on the contour of housing 302, a user will be able to determine by feel, how to position camera 300 to connect it with various pieces of equipment. Housing 302 may comprise any rigid material, such as for instance, stainless steel, that will protect the camera unit in a cost effective and durable manner.

Also illustrated in Figure 8 is coupling element 312. Coupling element 312 comprises coupling element housing 314, along with image guiding stem 316 and light guiding stem 318. The image guiding system extending through light guiding stem 318 comprises a bundle of coherent fiber optic cables to transmit reflected light from an area where illuminated light is be supplied to. Image guiding stem 316 and light guiding stem 318 are more readily seen as previously illustrated in Figure 6.

Coupling element housing 314 may comprise any suitable rigid material, such as for instance, stainless steel or a rigid plastic. Coupling element 312 is designed to detachably engage with image guiding receptacle 308 and light guiding receptacle 310.

While coupling element 312 illustrated in Figure 8 is not specifically shown connected to a particular instrument, it may be used in connection with practically any medical instrument using an illuminating system and an image guiding system. The coupling mechanism is universal and may be easily adapted depending upon the quality and resolution of image desired. It is further contemplated that although camera 300 is illustrating with image guiding cable 304 and light guiding cable 306 attached thereto, it is contemplated that wireless communication of generated camera data may be utilized.

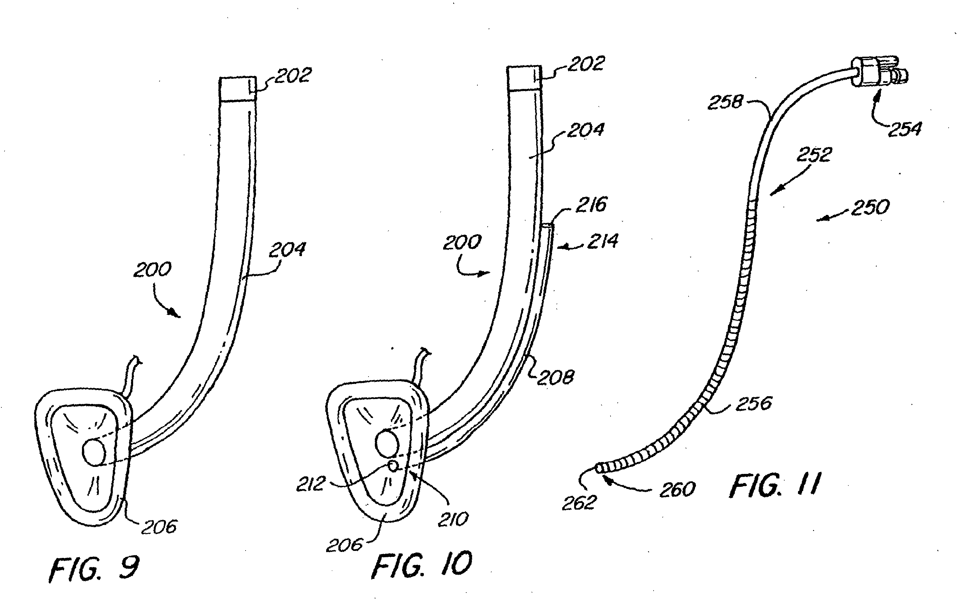

Figure 9 is an illustration of a Laryngeal Mask (LMA) 200 that includes a Supra Laryngeal Device (SLD) 202, a tube 204 and attached to a ventilator 206 to initiate air exchange. Typically SLD 202 comprises a flexible tube having an 18-20 mm OD. The LMA comprises a relatively small inflatable mask, which is insertable through the mouth and into the hypo-pharynx. Ventilator 206 is may be inflated so as to block the esophagus to avoid air-oxygen inflation of for example, the stomach. Ventilation may be provided through tube 204 connecting the opening of ventilator 206 into the trachea. Ventilator 206 may be adjusted according to the physiological parameters of the patient.

Figure 10 again illustrates LMA 200 including an attached guide tube or sheath 208. Sheath 208 may comprise a relatively flexible material and be formed substantially to the shape of tube 204 and may further be deformable according to tube 204. In this illustration, sheath 208 is show as attached to the exterior of tube 204; however, it is contemplated that an opening integral to tube 204 may also be provided. It is further contemplated that sheath 208 may be attachably and detachably connectable to LMA 200.

At a lower end portion 210 of sheath 208, an opening 212 is provided in ventilator 206 to which sheath 208 extends. At an upper end 214 of sheath 208 and opening 216 is provided.

Figure 11 illustrates one advantageous embodiment of the present invention depicting endotracheal device 250. In this embodiment, endotracheal device 250 comprises an outer housing 252 and a connector 254. Endotracheal device connector 254 is similar to coupling element 64 as previously described in connection with Figure 4 and will not be re-described here.

Outer housing 252 comprises a flexible portion 256 and a substantially rigid portion 258 within which a light and image guide (not shown) is maintained. The light and image guides are similar to those previously described in connection with alternative embodiments.

In this particular embodiment, approximately two-thirds the length of outer housing 252 is provided as flexible and approximately one-third is provided as substantially rigid, although these measurements may easily be adjusted based upon the application or other variables. It is contemplated that outer housing 252 may comprise stainless steel. At a distal end 260 of flexible portion 256 a window 262 seals outer housing 252.

Figure 12 illustrates the insertion of endotracheal device 250 into sheath 208. Also illustrated in securing clip 218 for securing endotracheal device to LMA 200. In this advantageous embodiment, securing clip 218 is attached to rigid portion 258 of outer housing 252 to maintain endotracheal device 250 in rigid relationship relative to LMA 200.

Also illustrated in Figure 12 is camera 300. Camera 300 may be any type of camera as previously discussed in connection with the alternative embodiments. Camera 300 is further provided with coupling element (camera connector) 312 that is engagable with endotracheal device connector 254. Coupling element 312 is similar to the light guiding system receptacle 52 and the image guiding system receptacle 48 described in connection with the previous Figures. Once camera 300 is rigidly attached to endotracheal device 250, it is contemplated that due to the robust connection between endotracheal connector 254 and coupling element 312, the user may grip the entire structure by camera 300, using camera 300 essentially as a handle for manipulation of the device.

It is contemplated that camera 300 may further comprise a light source for generating illuminating light to be transmitted to an area ahead of LMA 200. Reflected light is then transmitted back to camera 300 for generation of image data. Camera 300 may further be provided as a self-contained portable unit including a power source.

Camera 300 is coupled to video screen 274 by means of connection 276. It is contemplated that video screen 274 may be directly attached to camera 270 for local visualization of the area ahead of the LMA 200 during insertion. Alternatively, it is contemplated that video screen 274 may be remotely located from the patient and that connection 276 may comprise a wireless connection. This configuration is especially advantageous when, for example, a Medic Corpsman or first aid personnel need to intubate a patient in the field. Highly trained medical personnel that are not or cannot be at the location may visually see the intubation process on a video screen providing direction to the field medical personnel.

It is still further contemplated that both a local video screen and a remotely located video screen, via for example, an attachable telemetering unit, may be utilized for receiving images from camera 300 to facilitate intubation of the patient. This system is especially advantageous in for example a battlefield setting or at the scene of a disaster in which medical personnel with limited training can receive direct instruction from relatively highly trained medical personnel located remotely who are virtually transported to the scene and can provide highly specific instructions based on the image data they are receiving.

Connection 276 may comprise any type of wired or wireless connection for sending the image data to video screen 274. It is contemplated that connection 276 may further comprise a both a wired and a wireless connection, such that image data is transmitted to a transmitting unit, via for example a wired connection, which is then transmitted wirelessly to the remote video location. However, it is contemplated that many different communication schemes may be effectively utilized without deviating from the invention.

Although the invention has been described with reference to a particular arrangement of parts, features and the like, these are not intended to exhaust all possible arrangements or features, and indeed many other modifications and variations will be ascertainable to those of skill in the art.

## Claims

1. A medical device having
an endotracheal device (250) and a supra-laryngeal mask (200), wherein said endotracheal device (250) is attachable to a camera (300) and comprises
a light guide;
an image guide;
a first connector (254), said first connector (254) terminating the light guide and the image guide and being engageable with a second connector on the camera (300); and
an outer housing (252) enclosing said light and said image guides;
said outer housing (252) having a flexible portion (256) that may be bent by a user, and a rigid portion (258) that substantially maintains a rigid preformed shape;
and wherein said supra-laryngeal mask (200) comprises a sheath (208) attached to and substantially following a curvature of said supra-laryngeal mask (200), said sheath (208) having the endotracheal device (250) inserted therein;
and a securing clip (218) for affixing the endotracheal device (250) to the supra-laryngeal mask (200), wherein said securing clip (218) fastens between the supra-laryngeal mask (200) and the rigid portion (258) of the outer housing (252) of the endotracheal device (250).

2. The medical device of claim 1, **characterized in that** said first connector (254) comprises a two-stem connector, a first stem (318) terminating the light guide and a second stem (316) terminating the image guide.

3. The medical device of claim 1, **characterized in that** the supra-laryngeal mask (200) is disposable.

4. The medical device of claim 1, **characterized in that** the endotracheal device (250) further comprises a locking element (82) for securing the endotracheal device (250) to the camera (300).

5. The medical device of claim 1, wherein the flexible portion (256) of said outer housing (252) is greater than the rigid portion (258) of the outer housing (252).

6. The medical device of claim 1, **characterized in that** the outer housing (252) comprises stainless steel.

7. The medical device of claim 1, **characterized by** a camera (300) for generating image data, the camera (300) having a second connector that is engaged with said first connector (254) along a path of movement.

8. The medical device of claim 7, further **characterized by** a light source for generating illuminating light.

9. The medical device of claim 8, **characterized in that** said light source is located in the camera (300).

10. The medical device of claim 8, **characterized by** a video screen (274) for displaying of image data of the camera (300).

11. The medical device of claim 7, **characterized in that** a power source is located in the camera (300).

12. The medical device of claim 7, **characterized by** a transmitter for transmitting image data of said camera (300) to a remote location.

13. The medical device of claim 12, **characterized by**
a receiver for receiving the transmitted image data; and
a video screen (274) for display of the image data.

## Patentansprüche

1. Medizinische Vorrichtung,
mit einer endotrachealen Vorrichtung (250) und einer supralaryngealen Maske (200), wobei die endotracheale Vorrichtung (250) an eine Kamera (300) anbringbar ist und Folgendes aufweist, nämlich
einen Lichtleiter;
einen Bildleiter;
ein erstes Anschlussstück (254), wobei das erste Anschlussstück (254) den Lichtleiter und den Bildleiter abschließt und mit einem zweiten Anschlussstück an der Kamera (300) in Eingriff kommen kann; und
ein äußeres Gehäuse (252), das den Lichtleiter und den Bildleiter umschließt; wobei das äußere Gehäuse (252) einen flexiblen Abschnitt (256) aufweist, der durch einen Anwender abbiegbar ist, und einen steifen Abschnitt (258) aufweist, der im Wesentlichen eine steife vorgeformte Form beibehält;
und wobei die supralaryngeale Maske (200) eine Hülle (208) aufweist, die an der supralaryngealen Maske (200) angebracht ist und im Wesentlichen deren Krümmung folgt, wobei in der Hülle (208) die endotracheale Vorrichtung (250) eingeschoben ist;
und mit einem Befestigungsclip (218) zum Befestigen der endotrachealen Vorrichtung (250) an der supralaryngealen Maske (200), wobei der Befestigungsclip (218) zwischen der supralaryngealen Maske (200) und dem steifen Abschnitt (258) des äußeren Gehäuses (252) der endotrachealen Vorrichtung (250) verbindet.

2. Medizinische Vorrichtung nach Anspruch 1, **dadurch gekennzeichnet, dass** das erste Anschlussstück (254) ein Anschlussstück mit zwei Zapfen aufweist, wobei in einem ersten Zapfen (318) der Lichtleiter und in einem zweiten Zapfen (316) der Bildleiter endet.

3. Medizinische Vorrichtung nach Anspruch 1, **dadurch gekennzeichnet, dass** die supralaryngeale Maske (200) wegwerfbar ist.

4. Medizinische Vorrichtung nach Anspruch 1, **dadurch gekennzeichnet, dass** die endotracheale Vorrichtung (250) ferner ein Riegelelement (82) zum festen Anbringen der endotrachealen Vorrichtung (250) an der Kamera (300) aufweist.

5. Medizinische Vorrichtung nach Anspruch 1, wobei der flexible Abschnitt (256) des äußeren Gehäuses (252) größer ist als der steife Abschnitt (258) des äußeren Gehäuses (252).

6. Medizinische Vorrichtung nach Anspruch 1, **dadurch gekennzeichnet, dass** das äußere Gehäuse (252) Edelstahl enthält.

7. Medizinische Vorrichtung nach Anspruch 1, **gekennzeichnet durch** eine Kamera (300) zum Erzeugen von Bilddaten, wobei die Kamera (300) ein zweites Anschlussstück aufweist, das mit dem ersten Anschlussstück (254) längs eines Bewegungsweges in Eingriff steht.

8. Medizinische Vorrichtung nach Anspruch 7, ferner **gekennzeichnet durch** eine Lichtquelle zum Erzeugen von Beleuchtungslicht.

9. Medizinische Vorrichtung nach Anspruch 8, **dadurch gekennzeichnet, dass** die Lichtquelle in der Kamera (300) angeordnet ist.

10. Medizinische Vorrichtung nach Anspruch 8, **gekennzeichnet durch** einen Videobildschirm (274) zum Darstellen von Bilddaten der Kamera (300).

11. Medizinische Vorrichtung nach Anspruch 7, **dadurch gekennzeichnet, dass** in der Kamera (300) eine Energiequelle angeordnet ist.

12. Medizinische Vorrichtung nach Anspruch 7, **gekennzeichnet durch** einen Transmitter zum Übertragen von Bilddaten der Kamera (300) an eine fernab liegende Stelle.

13. Medizinische Vorrichtung nach Anspruch 12, **gekennzeichnet durch**
einen Empfänger zum Empfangen der übertragenen Bilddaten, und
einem Videobildschirm (274) zum Darstellen der Bilddaten.

## Revendications

1. Dispositif médical comportant
un dispositif endotrachéal (250) et un masque supra-laryngé (200), dans lequel ledit dispositif endotrachéal (250) peut être fixé à une caméra (300) et comprend
un guide de lumière,
un guide d'image,
un premier connecteur (254), ledit premier connecteur (254) terminant le guide de lumière et le guide d'image et pouvant être mis en contact avec un deuxième connecteur sur la caméra (300), et
un logement extérieur (252) enfermant lesdits guides de lumière et d'image,
ledit logement extérieur (252) comportant une partie flexible (256) qui peut être pliée par l'utilisateur et une partie rigide (258) qui conserve sensiblement une forme préformée rigide,
et dans lequel ledit masque supra-laryngé (200) comprend une gaine (208) fixée audit masque supra-laryngé (200) et suivant sensiblement une courbure de celui-ci, ladite gaine (208) ayant le dispositif endotrachéal (250) inséré dans celle-ci,
et une patte de fixation (218) destinée à fixer le dispositif endotrachéal (250) au masque supra-laryngé (200) où ladite patte de fixation (218) se fixe entre le masque supra-laryngé (200) et la partie rigide (258) du logement extérieur (252) du dispositif endotrachéal (250).

2. Dispositif médical selon la revendication 1, **caractérisé en ce que** ledit premier connecteur (254) comprend un connecteur à deux tiges, une première tige (318) terminant le guide de lumière et une deuxième tige (316) terminant le guide d'image.

3. Dispositif médical selon la revendication 1, **caractérisé en ce que** le masque supra-laryngé (200) est jetable.

4. Dispositif médical selon la revendication 1, **caractérisé en ce que** le dispositif endotrachéal (250) comprend en outre un élément de verrouillage (82) destiné à fixer le dispositif endotrachéal (250) à la caméra (300).

5. Dispositif médical selon la revendication 1, dans lequel la partie flexible (256) dudit logement extérieur (252) est plus grande que la partie rigide (258) du logement extérieur (252).

6. Dispositif médical selon la revendication 1, **caractérisé en ce que** le logement extérieur (252) comprend de l'acier inoxydable.

7. Dispositif médical selon la revendication 1, **caractérisé par** une caméra (300) destinée à générer des données d'images, la caméra (300) ayant un deuxième connecteur qui est amené en contact avec ledit premier connecteur (254) le long d'un trajet de déplacement.

8. Dispositif médical selon la revendication 7, **caractérisé en outre par** une source de lumière destinée à générer une lumière d'éclairage.

9. Dispositif médical selon la revendication 8, **caractérisé en ce que** ladite source de lumière est située dans la caméra (300).

10. Dispositif médical selon la revendication 8, **caractérisé par** un écran vidéo (274) destiné à afficher des données d'images de la caméra (300).

11. Dispositif médical selon la revendication 7, **caractérisé en ce qu'**une source d'alimentation est située dans la caméra (300).

12. Dispositif médical selon la revendication 7, **caractérisé par** un émetteur destiné à émettre des données d'images de ladite caméra (300) à un emplacement distant.

13. Dispositif médical selon la revendication 12, **caractérisé par**
un récepteur destiné à recevoir les données d'images émises, et
un écran vidéo (274) destiné à l'affichage des données d'images.
